# EUROPÄISCHE PATENTSCHRIFT

(11) **EP 0 794 943 B1**
(45) Veröffentlichungstag und Bekanntmachung des Hinweises auf die Patenterteilung: **15.09.1999**
(21) Anmeldenummer: 95941035.8
(22) Anmeldetag: 29.11.1995
(51) Int. Cl.: C07D 201/08

(54) **VERFAHREN ZUR HERSTELLUNG VON CAPROLACTAM**
METHOD FOR PREPARING CAPROLACTAM
PROCEDE DE PRODUCTION DE CAPROLACTAME

(30) Priorität: 03.12.1994 DE 4443125
(43) Veröffentlichungstag der Anmeldung: 17.09.1997
(73) Patentinhaber: BASF AKTIENGESELLSCHAFT, 67056 Ludwigshafen (DE)
(72) Erfinder: FUCHS, Eberhard, D-67227 Frankenthal (DE); ACHHAMMER, Günther, D-68309 Mannheim (DE)
(86) Internationale Anmeldenummer: EP9504680
(87) Internationale Veröffentlichungsnummer: WO9617826

(56) Entgegenhaltungen:
- EP-A- 0 150 295
- EP-A- 0 659 741
- DE-A- 4 319 134
- DE-A- 4 339 648
- FR-A- 2 029 540
- GB-A- 560 040
- GB-A- 560 100
- US-A- 2 301 964
- US-A- 2 357 484
- US-A- 4 625 023
- US-A- 4 628 085

## Beschreibung

Die vorliegende Erfindung betrifft ein verbessertes Verfahren zur Herstellung von Caprolactam durch Erhitzen von 6-Aminocapronsäurenitril in Gegenwart von heterogenen Katalysatoren und Wasser unter erhöhtem Druck.

Aus mehreren Patenten und Literaturstellen ist die Herstellung von 6-Aminocapronsäurenitril durch einseitige Hydrierung von Adipodinitril bekannt. Die Verwendung von Raney-Nickel wird beispielsweise in der DE 836 938, der DE 848 654 (beide BASF) und der US 5 151 543 (DuPont) beschrieben. Kinetische Untersuchungen finden sich bei C. Mathieu et al., Chem Eng. Sci., 47 (1992) 2289 - 2294.

In der US 4 628 085 (Allied) wird die Umsetzung von 6-Aminocapronsäurenitril mit Wasser in der Gasphase an einem speziellen sauren Kieselgel (Porasil A) bei 300°C beschrieben. Durch Verdünnung von 1,9% Substrat mit Wasser (14%), Ammoniak (6,3%) und Wasserstoff/Stickstoff kann Caprolactam bei quantitativem Umsatz in einer Selektivität über 95% erhalten werden, aber bereits innerhalb von 150 h findet durch Desaktivierung ein merklicher Umsatz- und Selektivitätsrückgang von je mindestens 5% statt.

Ein ähnliches Gasphasenverfahren wird auch in der US 4 625 023 (Allied) beschrieben. Hier wird ein hochverdünnter Gasstrom von 6-Aminocapronsäurenitril, Adipodinitril, Ammoniak, Wasser und Trägergas über ein Kieselgel und ein Kupfer/Chrom/Barium-Titanoxid-Katalysatorbett geleitet. Die Caprolactamselektivität beträgt 91% bei 85% Umsatz. Das Problem der Katalysatordesaktivierung wird diskutiert und Maßnahmen zu deren Verminderung durchgeführt, Angaben über deren Erfog fehlen jedoch.

Diese Verfahren haben beide den Nachteil einer raschen Katalysatordesaktivierung. Dieses Problem ist nicht gelöst.

In der US 2 245 129 (DuPont) wird die Herstellung linearer Polyamide durch Erhitzen einer 50%-igen wäßrigen Lösung von 6-Aminocapronsäurenitril auf 220°C für 20 h beschrieben. Über die Bildung von Caprolactam gibt es keine Hinweise.

Dagegen wird in der US 2 301 964 die nicht katalysierte Umsetzung von Aminocapronsäurenitril (als wäßrige Lösung) zu Caprolactam bei 285°C beschrieben. Die Ausbeute liegt deutlich unter 80% und man erhält einen nicht weiter beschriebenen Rückstand.

Die FR-A 2 029 540 beschreibt ein Verfahren zur Cyclisierung von 6-Aminocapronitril zu Caprolactam mittels Katalysatoren, wobei als Katalysatoren metallisches Zn oder Cu-Pulver oder Oxide, Hydroxide, Halogenide, Cyanide des Rubidiums, Bleis, Quecksilbers oder der Elemente mit einer Ordnungszahl 21 bis 30 oder 39 bis 48 Verwendung finden. Die beschriebenen Katalysatoren werden in diskontinuierlich betriebenen Rührautoklaven als Suspensionskatalysatoren eingesetzt. Caprolactam wird dabei in Ausbeuten bis zu 83 % erhalten. Die vollständige Abtrennung der Katalysatoren vom Wertprodukt Caprolactam bereitet jedoch Probleme, da Caprolactam mit den löslichen Bestandteilen der verwendeten Metalle Verbindungen bilden kann oder Feinstpartikel durch mechanisches Rühren entstehen können.

Es ist bekannt, 6-Aminocapronsäure, gelöst in Wasser (US 3 485 821), bei Temperaturen von 150 - 350°C zu Caprolactam zu cyclisieren.

Aus der DE-C 952 442 ist ein Verfahren bekannt, bei dem man durch hydrierende Aminierung von 5-Formylvaleriansäureestern in zwei Stufen Caprolactam neben Aminocapronsäureestern erhält.

In der US 3 988 319 (vgl. auch DE 2 535 689) wird ein Verfahren zur Cyclisierung von 6-Aminocapronsäure in Methanol oder Ethanol als Lösungsmittel beschrieben. Zur Vermeidung von Nebenreaktionen der 6-Aminocapronäure muß jedoch die Aminosäure so langsam in Lösung gebracht werden, daß sie sich nicht als Feststoff ansammelt. Hierfür sind Temperaturen von etwa 170°C notwendig. Weiterhin darf der Wassergehalt der Lösung nicht über 40 % ansteigen, da sich ansonsten offenkettige Polymere bilden. Das freiwerdende Reaktionswasser muß bei Wiederverwendung des Alkohols abgetrennt werden.

Dieselben Autoren, die auch die US 3 988 319 verfaßt haben, schreiben jedoch in Ind. Eng. Chem. Process Des. Dev., 17 (1978) 9 - 16, daß die Cyclisierung von 6-Aminocapronsäure in Wasser zu Caprolactam zu signifikanten Oligomerenmengen führt, wenn nicht bei Konzentrationen unter 13% und Temperaturen von rund 300°C gearbeitet wird.

A. Blade-Font beschreibt in Tetrahedron Lett., 21 (1980) 2443 - 2446 die Cyclisierung von 6-Aminocapronsäure als Suspension in Toluol in Gegenwart von Aluminiumoxid oder Kieselgel unter Auskreisen des Reaktionswassers. Zur vollständigen Desorption des Caprolactams muß der Katalysator mit Methylenchlorid/Methanol gewaschen und Polymere mit Diethylether ausgefällt werden. Die Ausbeute an Caprolactam beträgt nach 20 h Reaktionszeit 82% an Aluminiumoxid bzw. 75% an Kieselgel.

In der EP 271 815 wird die Cyclisierung von 6-Aminocapronsäureestern zu Caprolactam beschrieben, indem der Ester in einem aromatischen Kohlenwasserstoff gelöst, bei 100 bis 320°C cylisiert und gleichzeitig der abgespaltene Alkohol abgetrennt wird.

In der EP-A 376,122 wird die Cyclisierung von 6-Aminocapronsäureestern zu Caprolactam beschrieben, indem der Ester in einem aromatischen Kohlenwasserstoff gelöst und unter zusätzlicher Verwendung von Wasser bei Temperaturen von 230 bis 350°C, insbesondere bei 260 bis 340°C, cyclisiert wird.

Es ist bekannt Polyamid-6 zu Caprolactam zurückzuspalten. Die Spaltung erfolgt unter der Einwirkung saurer oder basischer Katalysatoren bei erhöhter Temperatur häufig unter Einwirkung von Wasserdampf, also im Niederdruckbereich. Chem. Ing. Techn. 45 (1973) 1510 beschreibt die technische Durchführung eines Spaltverfahrens mit überhitztem Wasserdampf, zur Aufarbeitung ist die Aufkonzentrierung einer Caprolactam/Wasser-Lösung nötig. In der EP 209021 wird die Spaltung in einem Aluminiumoxid-Wirbelbett durchgeführt. Die EP 529 470 setzt als Katalysator zur Polyamid-6-Spaltung Kaliumcarbonat zu und führt die Reaktion bei 250 bis 320°C unter gleichzeitigem Abdestillieren des Caprolactams im Vakuum durch.

Alle bisherigen Verfahren zur Spaltung von Polyamid-6 zu Caprolactam weisen als Nachteile die energetisch sehr aufwendige Abtrennung großer Wassermengen sowie das Entfernen von Katalysatoren wie Phosphorsäuren und deren Salze, Kaliumcarbonat oder Alkalimetalloxide auf. Im Falle der Gasphasenreaktionen wird das Polymer auf Temperaturen in der Regel im Bereich von 270 bis 400°C erhitzt und zusammen mit Wasser in einem Wirbelschichtreaktor gespalten. Nebenproduktbildungen und Deaktivierungen durch Verkleben der Katalysatorschüttung sind die Folge.

Aufgabe der vorliegenden Erfindung war es daher, ein verbessertes Verfahren zur Herstellung von Caprolactam, ausgehend von 6-Aminocapronsäurenitril, zur Verfügung zu stellen, das technisch praktikabel mit hoher Selektivität ohne die Probleme einer raschen Desaktivierung der eingesetzten Katalysatoren durchgeführt werden kann. Des weiteren sollte das Verfahren ohne nennenswerten Anfall an Leicht- und/oder Hochsiedern durchgeführt werden können.

Demgemäß wurde ein Verfahren zur Herstellung von Caprolactam durch Erhitzen von 6-Aminocapronsäurenitril in Gegenwart von heterogenen Katalysatoren und Wasser unter erhöhtem Druck gefunden, indem man
(a) 6-Aminocapronsäurenitril oder eine Mischung, enthaltend im wesentlichen 6-Aminocapronsäurenitril, sowie Wasser und einen leicht- oder einen hochsiedenden Alkohol in Gegenwart eines heterogenen Katalysators in einem Reaktor A erhitzt unter Erhalt einer Mischung I, dann
(b) Mischung I destilliert unter Erhalt von einer Kopffraktion, Caprolactam und einem Sumpf, wobei man, falls Mischung I Ammoniak enthält, diesen vor der Destillation entfernt, und anschließend
(c1) die Kopffraktion in den Reaktor A der Stufe (a) einspeist, wobei man die Kopffraktion gewünschtenfalls mit dem in Stufe (a) eingesetzten Alkohol und/oder Wasser und/oder 6-Aminocapronsäurenitril vor der Einspeisung in den Reaktor A vermischt, oder
(c2) die Kopffraktion, gewünschtenfalls mit dem Sumpf aus Stufe (b), in einen Reaktor B einspeist, wobei man die Kopffraktion gewünschtenfalls mit dem in Stufe (a) eingesetzten Alkohol und/oder Wasser und/oder 6-Aminocapronsäurenitril vor der Einspeisung in den Reaktor B vermischt, und anschließend analog zu Stufe (b) durch Destillation Caprolactam gewinnt, und entweder
(d1) den Sumpf aus Stufe (b) in den Reaktor A von Stufe (a) einspeist, oder
(d2) den Sumpf gegebenenfalls mit Wasser und gewünschtenfalls mit einem leicht- oder einem hochsiedenden Alkohol versetzt und dann analog zu Stufe (a) in einem weiteren Reaktor C erhitzt unter Erhalt eines Reaktionsaustrags, und aus dem erhaltenen Reaktionsaustrag Caprolactam durch Destillation gewinnt, oder
(d3) den Sumpf mit Wasser versetzt und ohne Zusatz eines Katalysators in einem Reaktor D erhitzt unter Erhalt eines Reaktionsaustrags, und aus dem erhaltenen Reaktionsaustrag Caprolactam durch Destillation gewinnt, oder
(d4) den mit Wasser und einer Base versetzten Sumpf in einem Reaktor E erhitzt unter Erhalt eines Reaktionsaustrags, und aus dem erhaltenen Reaktionsaustrag Caprolactam durch Destillation gewinnt.

Das erfindungsgemäß als Ausgangsmaterial dienende 6-Aminocapronsäurenitril erhält man üblicherweise durch Hydrierung von Adipodinitril nach einem bekannten Verfahren, beispielsweise beschrieben in DE-A 836 938, DE-A 848 654 oder US 5,151,543.

Man kann auch Mischungen in den Reaktor A einführen, die im wesentlichen 6-Aminocapronsäurenitril sowie Hexamethylendiamin, Adipodinitril und/oder Caprolactam enthalten können sowie hochsiedende Fraktionen ("Hochsieder") wie 6-Aminocapronsäureamid, 6-Aminocapronsäure, Polycaprolactam und Oligomere des Caprolactams und des weiteren 6-Aminocapronsäureester, die bei der Aufarbeitung des erfindungsgemäß hergestellten Caprolactams anfallen, wobei die 6-Aminocapronsäureester je nach Esterrest entweder leicht- oder hochsiedend sein können.

Erfindungsgemäß setzt man 6-Aminocapronsäurenitril mit Wasser um, wobei man bevorzugt pro mol 6-Aminocapronsäurenitril 0,01 bis 35 mol Wasser, besonders bevorzugt 1 bis 10 mol Wasser, einsetzt. Des weiteren setzt man erfindungsgemäß einen leicht- oder einen hochsiedenden Alkohol ein, wobei man bevorzugt eine Verdünnung von 1 bis 50 Gew.-% 6-Aminocapronsäurenitril wählt.

Leichtsiedende Alkohole sind solche, die einen Siedepunkt von höchstens 10°C bei einem Druck von 5 mbar unterhalb des Siedepunkts des Caprolactams haben wie C₁-C₁₀ -Alkanole, insbesondere Methanol, Ethanol, n-Propanol, i-Propanol, n-Butanol, i-Butanol, sek.-Butanol, besonders bevorzugt Methanol und Ethanol.

Hochsiedende Alkohole sind solche, die einen Siedepunkt von wenigstens 10°C bei einem Druck von 5 mbar oberhalb des Siedepunkts des Caprolactams haben wie Polyetherole, z.B. Tetraethylenglycol.

In einer weiteren Ausführungsform kann man dem Reaktionsgemisch von 0 bis 5, bevorzugt von 0,1 bis 2 Gew.-% Ammoniak und/oder Wasserstoff und/oder Stickstoff zusetzen.

Als heterogene Katalysatoren können beispielsweise verwendet werden: Saure, basische oder amphotere Oxide der Elemente der zweiten, dritten oder vierten Hauptgruppe des Periodensystems, wie Calciumoxid, Magnesiumoxid, Boroxid, Aluminiumoxid, Zinn-Oxid oder Siliciumdioxid als pyrogen hergestelltes Siliciumdioxid, als Kieselgel, Kieselgur, Quarz oder Mischungen derselben, weiterhin Oxide von Metallen der zweiten bis sechsten Nebengruppe des Periodensystems, wie Titanoxid, amorph, als Anatas oder Rutil, Zirkonoxid, Zinkoxid, Manganoxid oder Mischungen davon. Ebenfalls verwendbar sind Oxide der Lanthaniden und Aktiniden, wie Ceroxid, Thoriumoxid, Praseodymoxid, Samariumoxid, Seltenerd-Mischoxid, oder Mischungen davon mit zuvor genannten Oxiden. Weitere Katalysatoren können beispielsweise sein: Vanadiniumoxid, Nioboxid, Eisenoxid, Chromoxid, Molybdänoxid, Wolframoxid oder Mischungen davon. Mischungen der genannten Oxide untereinander sind ebenfalls möglich. Auch einige Sulfide, Selenide und Telluride wie Zink-Tellurid, Zinn-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. diese enthalten.

Weiterhin können Zeolithe, Phosphate und Heteropolysäuren sowie saure und alkalische Ionenaustauscher wie Naphion® als geeignete Katalysatoren eingesetzt werden.

Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Die Katalysatoren können je nach der Zusammensetzung des Katalysators als Vollkontakt oder Trägerkatalysator verwendet werden. So kann z.B. Titan-Dioxid als Titan-Dioxid-Strang oder als auf einen Träger in dünner Schicht aufgebrachtes Titandioxid eingesetzt werden. Zum Aufbringen von TiO₂ auf einen Träger wie Siliciumdioxid, Aluminiumoxid oder Zirkondioxid sind alle in der Literatur beschriebenen Methoden verwendbar. So kann eine dünne TiO₂-Schicht durch Hydrolyse von Ti-Organylen wie Ti-Isopropylat oder Ti-Butylat, oder durch Hydrolyse von TiCl₄ oder anderen anorganischen Ti-haltigen Verbindungen aufgebracht werden. Auch Titanoxid-haltige Sole sind verwendbar.

Erfindungsgemäß führt man die Reaktion in Stufe (a) bei einer Temperatur im Bereich von 100 bis 320, bevorzugt von 160 bis 280, besonders bevorzugt von 180 bis 260°C durch.

Üblicherweise führt man die Reaktion in Stufe (a) unter Druck durch, wobei man den Druck in der Regel im Bereich von 0,1 bis 50, bevorzugt von 0,5 bis 25 MPa wählt.

Die Reaktionsdauer im Reaktor A hängt im wesentlichen von den gewählten Verfahrensparametern ab und liegt beim kontinuierlich durchgeführten Verfahren im allgemeinen im Bereich von 1 bis 300, bevorzugt von 5 bis 120 min. Bei kürzeren Reaktionszeiten sinkt in der Regel der Umsatz, bei längeren Reaktionszeiten bilden sich nach bisherigen Beobachtungen vermehrt Oligomere, so daß größere Mengen an Oligomeren zur Spaltung zurückgeführt werden müßten.

Die Cyclisierung (Stufe (a)) führt man bevorzugt kontinuierlich in einem Reaktor A, vorzugsweise in einem Rohrreaktor, in Rührkesseln oder Kombinationen davon durch.

Die Cyclisierung (Stufe (a)) kann man auch diskontinuierlich durchführen. Die Reaktionsdauer liegt dann üblicherweise im Bereich von 30 bis 300 min.

Der Austrag aus Reaktor A ist erfindungsgemäß eine Mischung I, bestehend im wesentlichen aus Wasser, Alkohol, 6-Aminocapronsäureester, erhalten durch Reaktion von während der Reaktion gebildeter 6-Aminocapronsäure und dem eingesetzten Alkohol, Caprolactam, Ammoniak und hochsiedenden Verbindungen ("Hochsiedern") wie 6-Aminocapronsäureamid und Oligomeren von Caprolactam .

In Stufe (b) destilliert man erfindungsgemäß Mischung I nach üblichen Methoden unter Erhalt einer Kopffraktion, Caprolactam und einem Sumpf. Falls die Mischung I aus Stufe (a) Ammoniak enthält, entfernt man diesen erfindungsgemäß vor der Destillation. Die Entfernung des Ammoniaks kann nach üblichen Methoden vorgenommen werden, beispielsweise indem man destilliert oder einen Inertgasstrom durch Mischung I leitet.

Die Aufarbeitung der Mischung I kann man sukzessive oder gleichzeitig vornehmen, indem man beispielsweise in einer bevorzugten Ausführungsform das Wasser und gegebenenfalls einen damit azeotrop destillierenden leichtsiedenden Alkohol zuerst durch Destillation entfernt und den erhaltenen Rückstand einer oder mehreren weiteren Destillationen unterwirft, oder man destilliert Mischung I in einer einzigen Destillationskolonne.

In einer bevorzugten Ausführungsform führt man zunächst eine Destillation unter vermindertem Druck im Bereich von 10 bis 500 mbar, bevorzugt 50 bis 350 mbar aus unter Erhalt von Wasser und gegebenenfalls Alkohol sowie einem Destillationsrückstand, den man einer weiteren Destillation bei einer Temperatur im Bereich von 90 bis 220, vorzugsweise von 100 bis 160°C und einem Druck im Bereich von 0,01 bis 1, vorzugsweise von 0,5 bis 300 mbar unterwirft unter Erhalt von einer Kopffraktion, Caprolactam und einem Sumpf.

Die Kopffraktion besteht in der Regel aus im wesentlichen leichtsiedenden 6-Aminocapronsäurealkylestern, unumgesetztem 6-Aminocapronsäurenitril sowie - falls nicht schon separat abgetrennt - Wasser und dem eingesetzten Alkohol, falls er leichtsiedend ist.

Der Sumpf setzt sich in der Regel im wesentlichen aus hochsiedenden Anteilen wie Oligomeren des Caprolactams, 6-Aminocapronsäureamid, 6-Aminocapronsäure und, je nach eingesetztem Alkohol, hochsiedenden 6-Aminocapronsäureestern sowie, falls eingesetzt, hochsiedendem Alkohol zusammen.

Erfindungsgemäß führt man die Kopffraktion, gewünschtenfalls zusammen mit dem gegebenenfalls zuvor abgetrennten Wasser und Alkohol, in den Reaktor A zurück (Stufe (cl)), wobei man die Kopffraktion mit dem in Stufe (a) eingesetzten Alkohol und/oder Wasser und/oder 6-Aminocapronsäurenitril vor der Einspeisung in den Reaktor A vermischen kann.

Erfindungsgemäß kann man die in Stufe (b) erhaltene Kopffraktion fakultativ in einen Reaktor B einspeisen (Stufe (c2)), gewünschtenfalls mit dem Sumpf aus Stufe (b) und gewünschtenfalls zusammen mit dem gegebenenfalls zuvor abgetrennten Wasser und Alkohol, wobei man die Kopffraktion mit dem in Stufe (a) eingesetzten Alkohol und/oder Wasser und/oder 6-Aminocapronsäurenitril vor der Einspeisung in den Reaktor B vermischen kann. Die Reaktionsbedingungen in Reaktor B wählt man so, daß sie denen im Reaktor A entsprechen. Den Reaktionsaustrag aus Reaktor B arbeitet man analog zu Stufe (b) auf, wobei man Caprolactam in einer 5 oder mehreren Destillationsstufen erhält.

Den in Stufe (b) erhaltenen Sumpf kann man neben der zuvor genannten Variante in Stufe (c2) erfindungsgemäß auf vier weitere Arten aufarbeiten, wobei man entweder
in Stufe (d1) den Sumpf aus Stufe (b) in den Reaktor A von Stufe (a) einspeist, oder
in Stufe (d2) den Sumpf gegebenenfalls mit Wasser und gewünschtenfalls mit einem leicht- oder hochsiedenden Alkohol versetzt, bevorzugt mit der 0,1- bis 25-, besonders bevorzugt mit der 0,15- bis 15-fachen Gewichtsmenge an Wasser und vorzugsweise mit der 1- bis 25-, besonders bevorzugt mit der 3- bis 15-fachen Gewichtsmenge an Alkohol, und dann analog zu Stufe (a) in einem weiteren Reaktor C erhitzt unter Erhalt eines Reaktionsaustrags, und aus dem erhaltenen Reaktionsaustrag Caprolactam durch Destillation, bevorzugt analog zu Stufe (b), gewinnt, oder

(d3) den Sumpf mit Wasser versetzt, bevorzugt mit der 5- bis 25-, besonders bevorzugt mit der 7- bis 15-fachen Gewichtsmenge an Wasser, und ohne Zusatz eines Katalysators in einem Reaktor D erhitzt, wobei man die Reaktionsbedingungen vorzugsweise analog zu denen im Reaktor A wählt mit dem Unterschied, daß man die Temperatur im Bereich von 200 bis 350, vorzugsweise von 280 bis 320°C, und die Verweilzeit im Bereich von 5 bis 240 min wählt, unter Erhalt eines Reaktionsaustrags, und aus dem erhaltenen Reaktionsaustrag Caprolactam durch Destillation, bevorzugt analog zu Stufe (b), gewinnt, oder
(d4) den mit Wasser und einer Base versetzten Sumpf in einem Reaktor E erhitzt unter Erhalt eines Reaktionsaustrags, und aus dem erhaltenen Reaktionsaustrag Caprolactam durch Destillation, vorzugsweise analog zu Stufe (b), gewinnt, wobei man vorzugsweise den Sumpf unter vermindertem Druck im allgemeinen Bereich von 0,1 bis 50, vorzugsweise von 1 bis 10 mbar, in Gegenwart der Base, in der Regel von 1 bis 10, vorzugsweise von 1 bis 3 Gew.-%, im Reaktor E, bevorzugt ein Rohrreaktor, bei einer Temperatur im Bereich von 200 bis 400, vorzugsweise von 280 bis 320°C, erhitzt.

Prinzipiell kann man den Sumpf natürlich auch nach Methoden des Standes der Technik aufarbeiten, indem man ihn beispielsweise einem der gängigen Gasphasenverfahren oder einer der üblichen Aufarbeitungsmethoden mit sauren Katalysatoren unterwirft. Bevorzugt sind jedoch, wegen der Nachteile der Verfahren des Standes der Technik, die zuvor genannten erfindungsgemäßen Ausführungsformen (c2) und (d1) bis (d4).

Als Base setzt man bevorzugt Alkalimetallhydroxide und Alkalimetallcarbonate wie Natriumhydroxid, Kaliumhydroxid, Natriumcarbonat und Kaliumcarbonat oder eine Mischung davon ein, besonders bevorzugt Natrium- und/oder Kaliumhydroxid.

Der Vorteil des erfindungsgemäßen Verfahrens liegt darin, daß man Caprolactam technisch praktikabel mit hoher Selektivität und hoher Ausbeute ausgehend von 6-Aminocapronsäurenitril ohne die Probleme einer raschen Desaktivierung der eingesetzten Katalysatoren erhält, wobei keine nennenswerten Mengen an Leicht- und Hochsiedern anfallen.

### Beispiele

### Beispiel 1

(a) In einem auf 230°C geheizten Rohrreaktor von 20 ml Inhalt (Durchmesser 6 mm, Länge 710 mm), der mit Titanoxid (Anatas) in Form von 1,5 mm Strängen gefüllt war, wurden bei 100 bar 70 ml/h einer Lösung aus 10 Gew.-% 6-Aminocapronsäurenitril, 3,2 Gew.-% Wasser und Ethanol (Rest) eingeleitet.
   Die quantitative gaschromatographische Auswertung des Reaktionsaustrags ergab folgende Ausbeuten (ohne Ethanol und Wasser): 91% Caprolactam, 4% 6-Aminocapronsäureethylester sowie 1% 6-Aminocapronsäurenitril.
   Ein über 200 h gesammelter Produktstrom wurde von Ethanol und Wasser befreit und das so erhaltene Rohlactam destilliert. Dabei fielen 56 g leichtsiedene Anteile ("Leichtsieder") und 126 g hochsiedende Anteile ("Hochsieder") sowie 1232 g Caprolactam an. Der Leichtsiederanteil setzte sich im wesentlichen zusammen aus 6-Aminocapronsäureethylester und unumgesetztem 6-Aminocapronsäurenitril, der Hochsiederanteil aus Oligomeren.
(b) 126 g Oligomere, 56 g Leichtsieder (aus (a)) sowie 1200 g 6-Aminocapronsäurenitril wurden mit 445 g Wasser versetzt und mit Ethanol zu einer 10 gew.-%igen Lösung verdünnt. Diese Lösung wurde mit 70 ml/h erneut durch den Reaktor bei 230°C und 100 bar gepumpt. Die Ausbeuten des Austrags wurden mittels gaschromatischer Analyse (ber. ohne Ethanol und Wasser) bestimmt und betrugen: 87% Caprolactam, 3% 6-Aminocapronsäureethylester und 0,5% 6-Aminocapronsäurenitril.

Nach Destillation wurden aus dem zweiten Austrag 1182 g Caprolactam, 36 g Leichtsieder sowie 150 g Hochsieder erhalten. Insgesamt wurden aus 2600 g 6-Aminocapronsäurenitril 36 g rückführbare Leichtsieder, 150 g Hochsieder und 2432 g Caprolactam erhalten. Die Gesamtausbeute betrug 93%, die Selektivität 98%.

### Beispiel 2

(a) In einem auf 260°C geheizten Rohrreaktor von 20 ml Inhalt (Durchmesser 6 mm, Länge 710 mm), der mit Titanoxid (Anatas) in Form von 1,5 mm Strängen gefüllt war, wurden bei 200 bar 100 ml/h einer Lösung aus 10 Gew.-% 6-Aminocapronsäurenitril, 16,0 Gew.-% Wasser und Ethanol (Rest) eingeleitet.
   Die quantitative gaschromatographische Auswertung des Reaktionsaustrags ergab folgende Ausbeuten (ohne Ethanol und Wasser): 93% Caprolactam und 2% 6-Aminocapronsäureethylester.
   Ein über 200 h gesammelter Produktstrom wurde von Ethanol und Wasser befreit und das so erhaltene Rohlactam destilliert. Dabei fielen 55 g leichtsiedene Anteile ("Leichtsieder") und 140 g hochsiedende Anteile ("Hochsieder") sowie 1820 g Caprolactam an. Der Leichtsiederanteil setzte sich im wesentlichen zusammen aus 6-Aminocapronsäureethylester, der Hochsiederanteil aus Oligomeren.
(b) 140 g Oligomere, 55 g Leichtsieder (aus Beispiel 2a) sowie 2200 g 6-Aminocapronsäurenitril wurden mit 3830 g Wasser versetzt und mit Ethanol zu einer 10 gew.-%igen Lösung verdünnt. Diese Lösung wurde mit 100 ml/h erneut durch den Reaktor bei 260°C und 200 bar gepumpt. Die Ausbeuten des Austrags wurden mittels gaschromatischer Analyse (ber. ohne Ethanol und Wasser) bestimmt und betrugen: 91% Caprolactam und 2% 6-Aminocapronsäureethylester.

Nach Destillation wurden aus dem zweiten Austrag 2129 g Caprolactam, 57 g Leichtsieder sowie 196 g Hochsieder erhalten.

Insgesamt wurden aus 4200 g 6-Aminocapronsäurenitril 57 g rückführbare Leichtsieder, 196 g Hochsieder und 3945 g Caprolactam erhalten. Die Gesamtausbeute betrug 94%, die Selektivität 99%.

### Beispiel 3

(a) In einem auf 200°C geheizten Rohrreaktor von 20 ml Inhalt (Durchmesser 6 mm, Länge 710 mm), der mit Titanoxid (Anatas) in Form von 1,5 mm Strängen gefüllt war, wurden bei 100 bar 15 ml/h einer Lösung aus 10 Gew.-% 6-Aminocapronsäurenitril, 3,2 Gew.-% Wasser und Ethanol (Rest) eingeleitet.
   Die quantitative gaschromatographische Auswertung des Reaktionsaustrags ergab folgende Ausbeuten (ohne Ethanol und Wasser): 88% Caprolactam, 4% 6-Aminocapronsäureethylester und 4% 6-Aminocapronsäurenitril.
   Ein über 200 h gesammelter Produktstrom wurde von Ethanol und Wasser befreit und das so erhaltene Rohlactam destilliert. Dabei fielen 29 g leichtsiedene Anteile ("Leichtsieder") und 12 g hochsiedende Anteile ("Hochsieder") sowie 260 g Caprolactam an. Der Leichtsiederanteil setzte sich im wesentlichen zusammen aus 6-Aminocapronsäureethylester und unumgesetztem 6-Aminocapronsäurenitril, der Hochsiederanteil aus Oligomeren.
(b) 12 g Oligomere, 29 g Leichtsieder (aus Beispiel 3a) sowie 260 g 6-Aminocapronsäurenitril wurden mit 94 g Wasser versetzt und mit Ethanol zu einer 10 gew.-%igen Lösung verdünnt. Diese Lösung wurde mit 100 ml/h erneut durch den Reaktor bei 250°C und 200 bar gepumpt. Die Ausbeuten des Austrags wurden mittels gaschromatischer Analyse (ber. ohne Ethanol und Wasser) bestimmt und betrugen: 91% Caprolactam und 2% 6-Aminocapronsäureethylester.

Nach Destillation wurden aus dem zweiten Austrag 265 g Caprolactam, 83 g Leichtsieder sowie 25 g Hochsieder erhalten.

Insgesamt wurden aus 560 g 6-Aminocapronsäurenitril 83 g rückführbare Leichtsieder, 25 g Hochsieder und 525 g Caprolactam erhalten. Die Gesamtausbeute betrug 94%, die Selektivität 99%.

## Patentansprüche

1. Verfahren zur Herstellung von Caprolactam durch Erhitzen von 6-Aminocapronsäurenitril in Gegenwart von heterogenen Katalysatoren und Wasser unter erhöhtem Druck, dadurch gekennzeichnet, daß man
(a) 6-Aminocapronsäurenitril oder eine Mischung, enthaltend im wesentlichen 6-Aminocapronsäurenitril, sowie Wasser und einen leicht- oder einen hochsiedenden Alkohol in Gegenwart eines heterogenen Katalysators in einem Reaktor A erhitzt unter Erhalt einer Mischung I, dann
(b) Mischung I destilliert unter Erhalt von einer Kopffraktion, Caprolactam und einem Sumpf, wobei man, falls Mischung I Ammoniak enthält, diesen vor der Destillation entfernt, und anschließend
(c1) die Kopffraktion in den Reaktor A der Stufe (a) einspeist, wobei man die Kopffraktion gewünschtenfalls mit dem in Stufe (a) eingesetzten Alkohol und/oder Wasser und/oder 6-Aminocapronsäurenitril vor der Einspeisung in den Reaktor A vermischt, oder
(c2) die Kopffraktion, gewünschtenfalls mit dem Sumpf aus Stufe (b), in einen Reaktor B einspeist, wobei man die Kopffraktion gewünschtenfalls mit dem in Stufe (a) eingesetzten Alkohol und/oder Wasser und/oder 6-Aminocapronsäurenitril vor der Einspeisung in den Reaktor B vermischt, und anschließend analog zu Stufe (b) durch Destillation Caprolactam gewinnt, und entweder
(d1) den Sumpf aus Stufe (b) in den Reaktor A von Stufe (a) einspeist, oder
(d2) den Sumpf gegebenenfalls mit Wasser und gewünschtenfalls mit einem leicht- oder einem hochsiedenden Alkohol versetzt und dann analog zu Stufe (a) in einem weiteren Reaktor C erhitzt unter Erhalt eines Reaktionsaustrags, und aus dem erhaltenen Reaktionsaustrag Caprolactam durch Destillation gewinnt, oder
(d3) den Sumpf mit Wasser versetzt und ohne Zusatz eines Katalysators in einem Reaktor D erhitzt unter Erhalt eines Reaktionsaustrags, und aus dem erhaltenen Reaktionsaustrag Caprolactam durch Destillation gewinnt, oder
(d4) den mit Wasser und einer Base versetzten Sumpf in einem Reaktor E erhitzt unter Erhalt eines Reaktionsaustrags, und aus dem erhaltenen Reaktionsaustrag Caprolactam durch Destillation gewinnt.

2. Verfahren gemäß Anspruch 1, dadurch gekennzeichnet, daß man die Destillation in den Stufen (d2), (d3) und (d4) analog zu Stufe (b) durchführt unter Erhalt von Caprolactam, einer leichtsiedenden Fraktion und eines Sumpfes, mit der Maßgabe, daß man die leichtsiedende Fraktion in Stufe (b) zurückführt.

3. Verfahren nach den Ansprüchen 1 oder 2, dadurch gekennzeichnet, daß man eine Temperatur in den Reaktoren A bis D im Bereich von 100 bis 320°C einhält.

4. Verfahren nach den Ansprüchen 1 bis 3, dadurch gekennzeichnet, daß man einen Druck in den Reaktoren A bis D im Bereich von 0,1 bis 50 MPa einhält.

5. Verfahren nach den Ansprüchen 1 bis 4, dadurch gekennzeichnet, daß man je mol 6-Aminocapronsäurenitril 0,01 bis 35 mol Wasser einsetzt.

6. Verfahren nach den Ansprüchen 1 bis 5, dadurch gekennzeichnet, daß man eine Verweilzeit in den Reaktoren A bis D im Bereich von 1 bis 300 min einhält.

7. Verfahren nach den Ansprüchen 1 bis 6, dadurch gekennzeichnet, daß man einen Alkohol ausgewählt aus der Gruppe Methanol, Ethanol, n-, i-Propanol, n-, i-, sek.-Butanol oder Tetraethylenglykol verwendet.

## Claims

1. A process for the preparation of caprolactam by heating 6-aminocapronitrile in the presence of a heterogeneous catalyst and water under superatmospheric pressure, wherein
(a) 6-aminocapronitrile, or a mixture containing essentially 6-aminocapronitrile, and water and a low-boiling or a high-boiling alcohol are heated in the presence of a heterogeneous catalyst in a reactor A to give a mixture I,
(b) mixture I is then distilled to give a top fraction, caprolactam and a bottom product, ammonia being removed before the distillation if it is present in mixture I, and then
(c1)the top fraction is fed into the reactor A of stage (a), if desired the top fraction being mixed with the alcohol or water or 6-aminocapronitrile used in stage (a) before being fed into the reactor A, or
(c2) the top fraction, if desired with the bottom product from stage (b), is fed into a reactor B, the top fraction being mixed, if desired, with the alcohol or water or 6-aminocapronitrile used in stage (a) before being fed into the reactor B, and caprolactam is then obtained similarly to stage (b) by distillation, and either
(d1)the bottom product from stage (b) is fed into the reactor A of stage (a) or
(d2)if desired water and if desired a low-boiling or a high-boiling alcohol are added to the bottom product and the latter is then heated similarly to stage (a) in a further reactor C to give a reacted mixture, from which caprolactam is obtained by distillation, or
(d3) water is added to the bottom product, which is then heated in a reactor D without the addition of a catalyst to give a reacted mixture, and caprolactam is obtained from said reacted mixture by distillation, or
(d4) the bottom product to which water and a base have been added is heated in a reactor E to give a reacted mixture, from which caprolactam is obtained by distillation.

2. A process as claimed in claim 1, wherein the distillation in the stages (d2), (d3) and (d4) is carried out similarly to stage (b) to give caprolactam, a low-boiling fraction and a bottom product, with the proviso that the low-boiling fraction is recycled to stage (b).

3. A process as claimed in claim 1 or 2, wherein a temperature of from 100 to 320°C is maintained in the reactors A to D.

4. A process as claimed in any of claims 1 to 3, wherein a pressure of from 0.1 to 50 MPa is maintained in the reactors A to D.

5. A process as claimed in any of claims 1 to 4, wherein from 0.01 to 35 mol of water are used per mol of 6-aminocapronitrile.

6. A process as claimed in any of claims 1 to 5, wherein a residence time of from 1 to 300 minutes is maintained in the reactors A to D.

7. A process as claimed in any of claims 1 to 6, wherein an alcohol selected from the group consisting of methanol, ethanol, n-propanol, isopropanol, n-butanol, isobutanol, sec-butanol and tetraethylene glycol is used.

## Revendications

1. Procédé pour produire du caprolactame en chauffant du 6-aminocapronitrile en présence de catalyseurs hétérogènes et d'eau sous pression élevée, caractérisé en ce que
(a) on chauffe du 6-aminocapronitrile ou un mélange contenant essentiellement du 6-aminocapronitrile ainsi que de l'eau et un alcool à point d'ébullition faible ou élevé en présence d'un catalyseur hétérogène dans un réacteur A en obtenant un mélange I, puis
(b) on distille le mélange I en obtenant une fraction de tête, du caprolactame et un fond, en ayant éliminé l'ammoniac avant la distillation si le mélange I en contenait, et ensuite
(c1) on introduit la fraction de tête dans le réacteur A de l'étape (a), en mélangeant éventuellement la fraction de tête avec l'alcool mis en oeuvre dans l'étape (a) et/ou de l'eau et/ou le 6-aminocapronitrile avant de l'introduire dans le réacteur A, ou bien
(c2) on introduit la fraction de tête, éventuellement avec le fond provenant de l'étape (b), dans un réacteur B, en mélangeant éventuellement la fraction de tête avec l'alcool mis en oeuvre dans l'étape (a) et/ou de l'eau et/ou le 6-aminocapronitrile avant de l'introduire dans le réacteur B, puis on extrait par distillation le caprolactame de manière analogue à l'étape (b), et
(d1) soit on introduit le fond provenant de l'étape (b) dans le réacteur A de l'étape (a),
(d2) soit on mélange le fond éventuellement avec de l'eau et éventuellement avec un alcool à point d'ébullition faible ou élevé puis on le chauffe de manière analogue à l'étape (a) dans un réacteur supplémentaire C en obtenant un produit de réaction, et l'on extrait le caprolactame par distillation à partir du produit de réaction obtenu,
(d3) soit on mélange le fond avec de l'eau et on le chauffe dans un réacteur D sans ajouter de catalyseur, en obtenant un produit de réaction, et on extrait le caprolactame par distillation à partir du produit de réaction obtenu,
(d4) soit on chauffe le fond mélangé avec de l'eau et une base dans un réacteur E en obtenant un produit de réaction et l'on extrait le caprolactame par distillation à partir du produit de réaction obtenu.

2. Procédé selon la revendication 1, caractérisé en ce que l'on réalise la distillation dans les étapes (d2), (d3) et (d4) de manière analogue à l'étape (b) en obtenant le caprolactame, une fraction à faible point d'ébullition et un fond, avec la condition que l'on recycle la fraction à faible point d'ébullition dans l'étape (b).

3. Procédé selon les revendications 1 ou 2, caractérisé en ce que l'on maintient une température dans la plage de 100 à 320°C dans les réacteurs A à D.

4. Procédé selon les revendications 1 à 3, caractérisé en ce que l'on maintient une pression dans la plage de 0,1 à 50 MPa dans les réacteurs A à D.

5. Procédé selon les revendications 1 à 4, caractérisé en ce que l'on met en oeuvre 0,01 à 35 moles d'eau par mole de 6-aminocapronitrile.

6. Procédé selon les revendications 1 à 5, caractérisé en ce que l'on maintient une durée de séjour dans les réacteurs A à D dans la plage de 1 à 300 minutes.

7. Procédé selon les revendications 1 à 6, caractérisé en ce que l'on choisit un alcool dans le groupe formé par le méthanol, l'éthanol, le n-, i-propanol, le n-, i-, sec-butanol ou le tétraéthylèneglycol.
